# EUROPÄISCHE PATENTSCHRIFT

(11) **EP 3 127 613 B1**
(45) Veröffentlichungstag und Bekanntmachung des Hinweises auf die Patenterteilung: **24.07.2019**
(21) Anmeldenummer: 15002317.4
(22) Anmeldetag: 04.08.2015
(51) Int. Cl.: A61B 10/02, B01L 3/00, B01L 7/00, A61B 10/00

(54) **VORRICHTUNG ZUR ÜBERFÜHRUNG VON PROBENMATARIAL IN EINE FLÜSSIGKEIT**
DEVICE FOR THE TRANSFER OF SAMPLE MATERIAL INTO A LIQUID
DISPOSITIF DE CONTROLE DE MATERIAU D'ESSAI DANS UN LIQUIDE

(43) Veröffentlichungstag der Anmeldung: 08.02.2017
(73) Patentinhaber: Euroimmun Medizinische Labordiagnostika AG, 23560 Lübeck (DE)
(72) Erfinder: RICHTER, Carsten, 23560 Lübeck (DE)

(56) Entgegenhaltungen:
- EP-A1- 2 754 493
- US-A1- 2008 193 926
- US-A1- 2012 196 313
- US-A1- 2014 105 796

## Beschreibung

Die vorliegende Erfindung betrifft ein Aufbereitungsgefäß gemäß Anspruch 1 sowie ein Verfahren umfassend die Schritte Einführen eines Probenentnahmestäbchens, aufweisend eine zu untersuchende Probe in das erfindungsgemäße Aufbereitungsgefäß, bis das die Probe aufweisende Ende des Probenentnahmestäbchen auf den Gefäßboden aufsetzt, Abbrechen des Probenentnahmestäbchens, optional Verschließen des Aufbereitungsgefäßes und Kontaktieren des Endes des Probenentnahmegefäßes, an dem sich die zu untersuchende Probe befindet, mit einer Flüssigkeit unter Freisetzung der Probe in die Flüssigkeit.

Bei vielen diagnostischen, labormedizinischen oder sonstigen analytischen Untersuchungen bedient man sich chemischer Reaktionen und/oder physikalischer Nachweisverfahren in einer flüssigen Phase, das zu untersuchende Probenmaterial liegt unmittelbar nach der Gewinnung aber in fester oder inhomogener Form vor. Es muss vor der Untersuchung daher über eine geeignete Aufarbeitung in die flüssige Phase überführt werden.

So werden für mikrobiologische und molekulardiagnostische Tests beispielsweise Abstriche von Schleimhäuten oder Stuhlproben des Patienten entnommen und das Probenmaterial in wässrigem Puffer aufgelöst und optional chemisch oder physikalisch aufgeschlossen. Das aufgeschlossene Material kann dann für die Analyse verwandt werden. Beispielsweise können Abstriche von Rachen und Mund von Patienten gewonnen, die auf multiresistente Keime zu testen sind, und anschließend mittels Polymerase-Kettenreaktionen diagnostisch untersucht werden, die dem Fachmann geläufig sind. Ähnlich kann man Proben aus Lebensmitteln, zu untersuchenden Böden oder Materialien aufbereiten und analysieren.

Der Arbeitsaufwand bei dieser weitgehend manuell durchgeführten Aufbereitung, die mehrere Schritte umfasst, ist enorm und fällt insbesondere ins Gewicht, wenn eine Vielzahl von Proben parallel zu untersuchen ist. Zusätzlicher Aufwand ist erforderlich, wenn die nachfolgende diagnostische oder analytische Untersuchung in einem anderen Gefäß als die Aufbereitung stattfindet, weil das aufgeschlossene Material zunächst in eine Pipette aufgenommen und dann in das Gefäß für die Untersuchung überführt werden muss.

Der Stand der Technik beschreibt eine Reihe von technischen Lösungen, mit denen eine standardmäßige Gewinnung einer Probe mittels eines Probenentnahmestäbchens möglich ist. Dazu wird die Probe mit einem Ende des Probenentnahmestäbchens aufgenommen, beispielsweise durch Aufwischen. Das Probeentnahmestäbchen wird anschließend in ein Plastikröhrchen eingeführt, das eine Flüssigkeit enthält. Das Stäbchen wird anschließend über den oberen Rand des Plastikröhrchens abgebrochen, und die Probe durch Bewegen des abgebrochenen Stäbchens in dem verschlossenen Röhrchen in die Flüssigkeit im Röhrchen überführt.

Diese Lösung weist jedoch eine Reihe von technischen Nachteilen auf. Zunächst wird das Probenentnahmestäbchen über den oberen, meist runden Rand des Plastikröhrchens abgebrochen. Selbst wenn dies unter Einsatz beider Hände geschieht, besteht die Gefahr, dass das Stäbchen dabei verrutscht, wobei es mit der Probe aus dem Röhrchen geschleudert werden kann. Außerdem besteht die Gefahr, dass die Flüssigkeit, die gesundheitsschädliche Inhaltsstoffe aufweisen kann, unter Freisetzung von Probenmaterial in die Umgebung aus dem Röhren spritzt, im schlimmsten Fall ins Gesicht des Anwenders. Die US2014/105796A1 ist Bestandteil des Standes der Technik.

Die der vorliegenden Erfindung zu Grunde liegende Aufgabe besteht darin, die genannten Probleme der im Stand der Technik beschriebenen Vorrichtungen zu lösen. Insbesondere soll eine Vorrichtung zum Überführen einer mit einem Probenentnahmestäbchen aufgenommenen Probe in eine wässrige Lösung bereitgestellt werden, wobei möglichst wenig Schritte, bevorzugt möglichst wenig manuell auszuführende Schritte erforderlich sind. Für den Fall, dass die Nachweisreaktion in einem automatisierungsfähigen Laborgerät durchgeführt wird, soll das Verfahren mit möglichst wenigen Schritten außerhalb des Gerätes auskommen.

Weiterhin soll erfindungsgemäß eine Vorrichtung bereitgestellt werden, die eine möglichst reproduzierbare, sichere und möglichst wenig unfall- und probenverlustanfällige Überführung einer Probe in eine Lösung gestattet.

Schließlich soll erfindungsgemäß eine Vorrichtung zur Prozessierung von biologischen Proben bereitgestellt werden, bei der die Gefahr, dass die Umwelt bzw. das prozessierende Labor durch die Probe kontaminiert wird, möglichst gering ist.

Diese und weitere Aufgaben werden durch den Gegenstand der vorliegenden Anmeldung und insbesondere auch durch den Gegenstand der beigefügten unabhängigen Ansprüche gelöst, wobei sich Ausführungsformen aus den Unteransprüchen ergeben.

Beschrieben wird ein Probenentnahmestäbchen umfassend ein Stäbchen, das durch mechanischen Druck an der Stelle abbricht, auf die der Druck ausgeübt wird, in Form einer Sollbruchstelle, sowie an einem Ende des Stäbchens eine zu untersuchende Probe.

Das Ende, an dem die Probe lokalisiert ist, ist mit einem probenaufnehmenden Material ausgestattet.

Das Ende, an dem die Probe lokalisiert ist, ist verdickt.

Die der Erfindung zu Grunde liegende Aufgabe wird in einem ersten Aspekt gelöst durch ein Aufbereitungsgefäß umfassend die Merkmale des Anspruchs 1.

In einer ersten nicht erfindungsgemäßen Ausführungsform liegt die erste Kante vertikal unterhalb der Öffnung.

In einer zweiten nicht erfindungsgemäßen Ausführungsform ist die Öffnung mit einem Deckel verschließbar, optional tatsächlich damit verschlossen.

In einer dritten bevorzugten Ausführungsform ist das Gefäß konisch ausgeführt.

Auf gleicher Höhe gegenüber von der ersten Kante liegt erfindungsgemäß eine zweite Kante, die mit der ersten Kante einen Spalt bildet.

Der Spalt verengt sich in Richtung vom Inneren der Öffnung nach außen hin.

Der Gefäßboden weist erfindungsgemäß einen oder mehr als einen Steg zum Aufsetzen des Endes des Probenentnahmestäbchens auf den Gefäßboden auf.

In einer Ausführungsform ist die erste und optional, die zweite Kante zugespitzt.

In einer bevorzugten Ausführungsform schließen die Kante und der Spalt an eine Einführöffnung zum Einführen des Endes des Probenentnahmestäbchens an, wobei der Durchmesser der Einführöffnung an der breitesten Stelle wenigstens so groß, bevorzugt größer ist als der Durchmesser des Spaltes an dessen breitester Stelle und wobei die Einführöffnung so ausgestaltet ist, dass das Probenentnahmestäbchen durch die Einführöffnung bis zum Gefäßboden in das Aufbereitungsgefäß eingeführt werden kann, wobei die Einführöffnung vertikal bevorzugt auf gleicher Höhe liegt wie die Kante.

In einer bevorzugten Ausführungsform weist das Aufbereitungsgefäß einen Auslass- und/oder Einlasskanal auf und ist optional in einen Laborchip integriert.

Die der Erfindung zu Grunde liegende Aufgabe wird in einem zweiten Aspekt gelöst durch ein Verfahren umfassend die Schritte
a) Einführen des Probenentnahmestäbchens, aufweisend eine zu untersuchende Probe in das erfindungsgemäße Aufbereitungsgefäß, bis das die Probe aufweisende Ende des Probenentnahmestäbchen auf den Gefäßboden aufsetzt,
b) Abbrechen des Probenentnahmestäbchens,
c) optional Verschließen des Aufbereitungsgefäßes und
d) Kontaktieren des Endes des Probenentnahmestäbchens, an dem sich die zu untersuchende Probe befindet, mit einer Flüssigkeit unter Freisetzung der Probe in die Flüssigkeit.

In einer ersten bevorzugten Ausführungsform des zweiten Aspekts wird das Probenentnahmestäbchen in Schritt a) zum nachfolgenden Abbrechen bis zur engsten Stelle des Spalts geführt.

Bei dem Probeentnahmestäbchen handelt es sich um ein längliches Stäbchen mit einem Stiel und zwei Enden, das so ausgestaltet ist, dass es am einen Ende festgehalten werden kann, um mit dem anderen Ende des Stäbchens die zu untersuchende Probe aufzunehmen und anschließend bis zum Boden eines geeigneten Aufbereitungsgefäßes zu bringen. Entsprechend ist das Ende, an dem die zu untersuchende Probe aufgenommen wird, in geeigneter Weise zur Probenaufnahme ausgestaltet, insbesondere durch eine geometrische Form mit Verdickung, beispielsweise eine birnenförmige Verdickung, ein Pinsel, eine Bürste oder dergleichen. Das Material an dem Ende, mit dem die Probe aufzunehmen ist, ist zur Aufnahme der jeweiligen Probe gewählt, beispielsweise als absorptionsstarker Wattekopf zur Aufnahme einer Flüssigkeit.

Bei der Probe kann es sich um eine beliebige Probe handeln, die möglicherweise oder tatsächlich eine zu charakterisierende oder nachzuweisende Komponente aufweist. Denkbar sind Proben mit tierischem, insbesondere menschlichen, pflanzlichen Material, Boden-, Wasser-, Lebensmittelproben oder dergleichen. Sie können auf chemische, physikalische oder biologische Eigenschaften untersucht werden. In einer besonders bevorzugten Ausführungsform handelt es sich um eine menschliche Probe, die diagnostisch, bevorzugt auf Pathogene zu untersuchen ist.

Für die Untersuchung muss die Probe in eine Flüssigkeit, bevorzugt wässrige Phase überführt und anschließend mittels biologischer, chemischer oder physikalischer Verfahren untersucht werden. Für den Fall, dass die Probe auf die Anwesenheit oder Zahl von bestimmten Mikroben, insbesondere Pathogene zu untersuchen ist, bietet sich eine Untersuchung über eine Polymerase-Kettenreaktion (PCR-Reaktion) an. Dazu werden in der Probe enthaltene Zellen aufgeschlossen, beispielsweise über einen lysierenden Puffer und/oder Ultraschall, wodurch die in den Zellen enthaltenen Nukleinsäuren in die wässrige Phase übertreten und für die chemische Nachweisreaktion zur Verfügung stehen.

Das Probenentnahmestäbchen weist einen Stiel aus einem Material aus, das abbricht, wenn genügend mechanischer Druck ausgeübt wird. Es kann sich um ein Holzstäbchen oder ein abbrechbares Plastikstäbchen handeln. An der Stelle des Stiels entlang der Längsachse, an dem der Bruch erfolgen soll, ist bevorzugt eine Sollbruchstelle vorgesehen, die bewirkt, dass der Bruch bevorzugt an dieser Stelle erfolgt, beispielsweise durch Einarbeiten einer Kerbe.

Erfindungsgemäß wird das Probenentnahmestäbchen, das an einem der beiden Enden die zu untersuchende Probe aufweist, zusammen mit einem Aufbereitungsgefäß benutzt. Probenentnahmestäbchen und Aufbereitungsgefäß sind von ihrer Größe und Gestalt aufeinander abgestimmt, so dass das Probenentnahmestäbchen mit dem die Probe aufweisenden Ende derart voran in das Aufbereitungsgefäß eingeführt werden kann, dass das Ende mit der Probe in Richtung des Gefäßbodens des Aufbereitungsgefäßes weist und schließlich auf den Gefäßboden aufsetzt.

Das Aufbereitungsgefäß ist weiterhin so ausgestaltet, dass eine Flüssigkeit, besonders ein wässriger Puffer, eingeführt und mit der Probe in Kontakt gebracht werden kann. Es besteht aus einem geeigneten flüssigkeitsdichten, chemisch ausreichend inerten Material, beispielsweise Kunststoff. Optional weist das Aufbereitungsgefäß einen Kanal auf, über den eine Flüssigkeit ein- und ausgelassen werden kann, oder einen Einlass- und, davon separat, einen Auslasskanal. Alternativ kann Flüssigkeit auch über die Öffnung eingefüllt und wieder entfernt werden. Der Kanal, bevorzugt der Auslasskanal, mündet bevorzugt in den Gefäßboden, so dass im Gefäß enthaltene Flüssigkeit bei aufrechtem Stand des Gefäßes unterstützt durch die Schwerkraft abfließen kann.

Das Aufbereitungsgefäß weist eine Öffnung auf, die bevorzugt an der Oberseite des Gefäßes angeordnet ist und durch einen bevorzugt hochstehenden Rahmen abgegrenzt sein kann. Bevorzugt ist die Öffnung mit einem Deckel verschließbar, und in diesem Fall ist der Rahmen derart ausgestaltet, dass der Deckel zum Verschluss daran angebracht werden kann. Beispielsweise kann der Rahmen innen ein Gewinde zum Aufschrauben eines Deckels aufweisen. Der Deckel kann optional über eine Verbindung lösbar oder unlösbar mit dem Gefäß verbunden sein, unabhängig davon, ob er die Öffnung verschließt oder nicht.

Das Aufbereitungsgefäß weist eine erste Kante auf, die bevorzugt im Wesentlichen parallel zur durch die Öffnung vorgegebenen Ebene angeordnet ist. Sie ist so positioniert, dass ein durch die Öffnung eingeführtes Probenentnahmeröhrchen an seinem Stiel an der Kante abgebrochen werden kann, wenn es so weit in das Gefäß eingeführt ist, dass sein die Probe aufweisendes Ende auf den Gefäßboden aufsetzt. Bevorzugt ist die Kante so angeordnet, dass sie in die Mitte der Öffnung weist. Das Aufbereitungsgefäß mit der Kante ist bevorzugt so ausgestaltet, dass das eingeführte Probenentnahmestäbchen durch das Aufsetzen am Gefäßboden einerseits und das Drücken auf die Kante andererseits so fixiert ist, dass die geometrischen Freiheitsgrade beim Abbrechen gegenüber einem Gefäß ohne Kante verringert sind und ein Verrutschen erschwert wird. Besonders bevorzugt weist das Probenentnahmestäbchen eine Sollbruchstelle an der Stelle auf, die die Kante berührt, wenn das Probenentnahmestäbchen in das Aufbereitungsgefäß eingeführt ist, bis das Ende mit der Probe auf den Gefäßboden aufsetzt.

Das Aufbereitungsgefäß weist neben der ersten Kante eine zweite Kante auf, die vertikal auf gleicher Höhe wie die erste Kante angeordnet ist, so dass beide Kanten einen Spalt bilden. Der Spalt verengt sich vom Inneren der Öffnung nach außen. In einer bevorzugtesten Ausführungsform weist der Spalt an seinem Ende am Außenrand der Öffnung eine Breite auf, die so ausgestaltet ist, dass das Probenentnahmestäbchen, wenn es in diesen Spalt bis zum Ende am Außenrand eingeführt wird, durch den Spalt einerseits und das Aufsetzen des Endes mit der Probe auf den Gefäßbodens andererseits so fixiert ist, dass eine Bewegung des Probenentnahmestäbchens ausschließlich in Richtung der Mitte der Öffnung möglich ist, also direkt zurück. Ein auf diese Weise fixiertes Probenentnahmestäbchen kann abgebrochen werden, ohne dass die Gefahr eines Verrutschens besteht. Dementsprechend ist das Abbrechen besonders einfach und die Gefahr des Herausspritzens von Flüssigkeit und/oder Probenmaterial minimal.

Die erste und zweite Kante sind vertikal unterhalb der Höhe der Öffnung angeordnet. Auf diese Weise wird das Probenentnahmestäbchen in einer Länge abgebrochen, die die vertikale Höhe des Gefäßes bis zur Öffnung unterschreitet. Dies gewährleistet, dass das Gefäß enthaltend das abgebrochene Probenentnahmestäbchen bequem mit dem Deckel verschlossen werden kann.

In einer bevorzugten Ausführungsform ist das Aufbereitungsgefäß konisch ausgeführt, d.h. an seinem Boden ist vertikal gesehen der Punkt mit dem geringsten Umfang. Optional weist das Aufbereitungsgefäß am Boden einen Zylinder auf, der an die Form des Endes des Probenentnahmestäbchens mit der Probe angepasst ist, welches besonders bevorzugt verdickt ist, und über diesen Zylinder, der am Ende der Verdickung abschließt, weitet sich das Probenentnahmegefäß auf. Auf diese Weise ist das Probenentnahmestäbchen beim Abbrechen besonders gut fixiert, während die zum Bedecken der Probe notwendige Flüssigkeitsmenge minimiert ist.

Bevorzugt weist das Aufbereitungsgefäß nahe des Bodens, bevorzugt im unteren Drittel, bevorzugt Viertel, bevorzugt Fünftel, bevorzugt Zehntel aus der Wand des Aufbereitungsgefäßes ragend wenigstens eine weitere Kante, bevorzugt symmetrisch auf gleicher Höhe angeordnet eine Gruppe umfassend wenigstens zwei weitere Kanten oder eine weitere durchgehende Kante auf. Das Probenentnahmestäbchen kann beim Einführen oder danach an dieser wenigstens einen Kante abgewischt werden, wobei die Probe in das Gefäß überführt wird.

Jede der erfindungsgemäß vorgesehenen Kanten kann zugespitzt sein, um das Abbrechen des Probeentnahmestäbchens oder das Abwischen zu erleichtern.

Das Aufbereitungsgefäß weist am Gefäßboden einen oder mehr als einen Steg auf. Dieser ist so angeordnet, dass er ein Verrutschen des am Gefäßboden aufsetzenden Endes des Probenentnahmestäbchens verhindert. Weiterhin kann er so angeordnet sein, dass er über einem am Boden des Gefäßes positionierten Kanal liegt. Auf dieses Weise wird ein Eindringen und Verstopfen des Kanals durch das Ende des Probenentnahmestäbchens verhindert.

In einer weiteren bevorzugten Ausführungsform schließt die Kante oder optional, sofern vorhanden, der Spalt derart an eine Einführöffnung zum Einführen des Endes des Probenentnahmestäbchens mit der Probe an, dass ein bis zum Aufsetzen auf den Gefäßboden eingeführtes Probenentnahmestäbchen lateral aus der Einführöffnung in den Spalt geschoben werden kann. Die Einführöffnung ist eine bevorzugt vertikal auf gleicher Höhe wie die Kante liegende, bevorzugt mit Hinblick auf den Umfang gegenüber der Öffnung verengte weitere Öffnung, die nach dem Passieren der Öffnung das weitere Einführen des Probenentnahmestäbchens kanalisiert. Der Durchmesser der Einführöffnung an ihrer breitesten Stelle ist wenigstens gleich groß oder bevorzugt größer als der Durchmesser des Spalts an seiner breitesten Stelle.

Das Aufbereitungsgefäß kann in einen Laborchip integriert sein. Das bedeutet, dass das Aufbereitungsgefäß eine von mehreren Kammern das Laborchips darstellt, die mit einander über Kanäle derart verbunden sind, dass je nach Bedarf eine Flüssigkeit von einer Kammer in eine andere übertragen werden kann. Beispielsweise kann die Flüssigkeit nach Aufnahme der Probe vom Probenentnahmestäbchen in eine weitere Kammer übertragen werden, in der die Probe in aufgereinigter Form oder mit einem Reagenz zur Reaktion gebracht wird. In einer bevorzugten Ausführungsform wird unter dem Begriff "Laborchip", wie hierin verwendet, ein abgeschlossenes System verstanden, das alle Analyseschritte von der Vorbereitung einer Probe bis zu deren Auswertung in einem integrierten, bevorzugt multiparameterfähigen System vereint. In einer bevorzugten Ausführungsform weist der Laborchip mehrere Kammern auf, die eine zu untersuchende Probe und für diagnostische Reaktionen geeignete Reagenzien enthalten, wobei bevorzugter neben der Probe für die Nachweisreaktion keine weiteren Materialien in den vorgefertigten Laborchip eingebracht werden müssen. In einer besonders bevorzugten Ausführungsform handelt es sich bei dem Laborchip um ein Einwegartikel.

Das erfindungsgemäße Verfahren sieht vor, dass das Ende des Probenentnahmestäbchens, an dem sich die zu untersuchende Probe befindet, mit einer Flüssigkeit kontaktiert wird, wobei die Probe in die Flüssigkeit freigesetzt wird. Dies geschieht nach dem Einführen des Probenentnahmestäbchens. Bevorzugt wird vorher das Probenentnahmestäbchen abgebrochen und der Deckel verschlossen. Bevorzugt handelt es sich bei der Flüssigkeit um einen wässrigen Puffer. Das Freisetzen kann durch mechanisches manuelles Führen oder, nach dem Verschließen, durch Schütteln, manuell oder mit Hilfe einer geeigneten mechanischen Vorrichtung wie einem Vortex, erleichtert werden. Alternativ wird die Flüssigkeit derart eingeleitet, dass eine Strömung entsteht, die das Freisetzen der Probe vorantreibt. Die Flüssigkeit kann auch mehrfach ein- und wieder ausgeleitet werden, um das Freisetzen zu unterstützen. Für den Fall, dass die Probe Zellen enthält, deren Inhalte untersucht werden sollen, kann es sich bei der Flüssigkeit um einen wässrigen Lysepuffer handeln. Optional wird das Aufbereitungsgefäß zusätzlich oder alternativ mit Ultraschall behandelt, um die Zellen aufzuschließen, oder es enthält ein Mittel zum mechanischen Aufschließen der Probe, beispielsweise Kügelchen aus Keramik oder Metall. In einer an das Aufbereitungsgefäß angeschlossenen Kammer können die Zellinhalte, insbesondere Nukleinsäuren, anschließend aufgereinigt und einer analytischen Reaktion unterzogen werden. In einer weiteren besonders bevorzugten Ausführungsform kann es sich bei der Flüssigkeit um einen Szinillationspuffer handeln, der Messung der Radioaktivität geeignet ist, die die Probe aufweist.

Im Folgenden wird die Erfindung unter Bezugnahme auf die Figuren anhand von Ausführungsbeispielen erläutert. Die beschriebenen Ausführungsformen sind in jeder Hinsicht lediglich beispielhaft und nicht als einschränkend zu verstehen, und verschiedene Kombinationen der angeführten Merkmale sind vom Umfang der Erfindung umfasst.

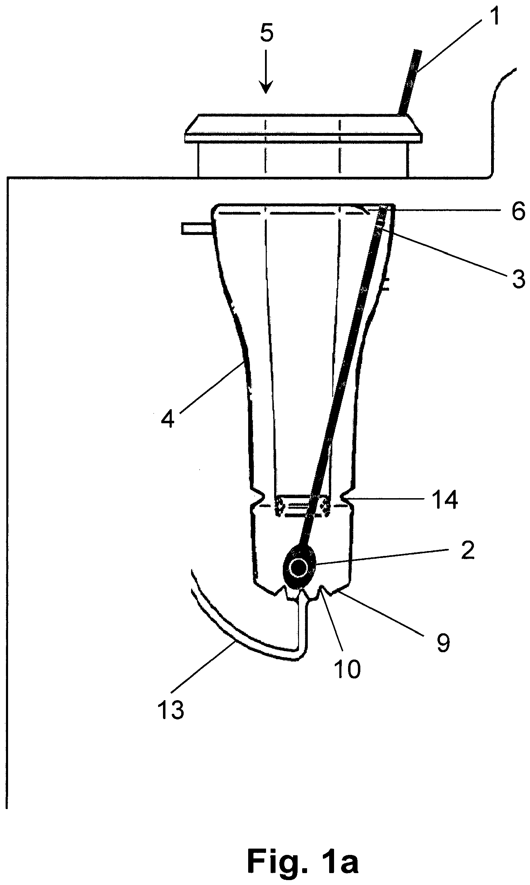

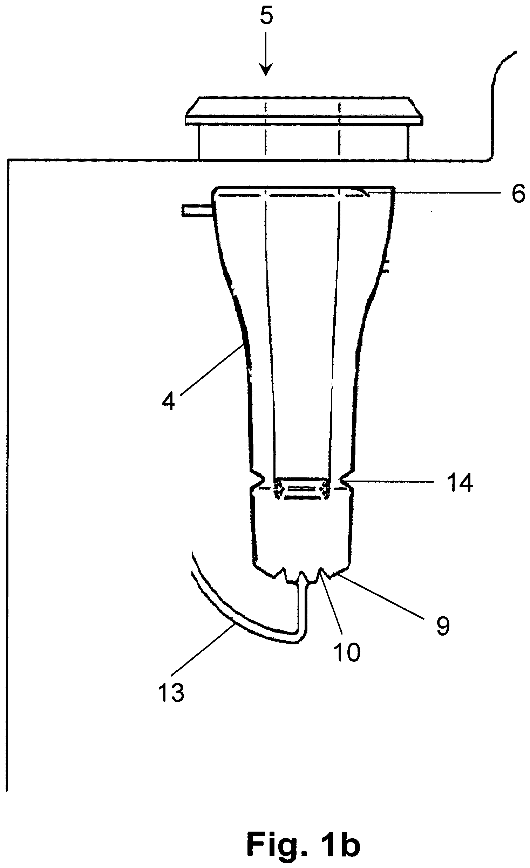

**Fig. 1a** zeigt eine nichterfindungsgemäße Vorrichtung mit einem Probenentnahmestäbchen (1), das ein Ende mit einer zu untersuchenden Probe (2) und eine Sollbruchstelle (3) aufweist und mit dem die Probe aufweisenden Ende voran soweit in ein erfindungsgemäßes Aufbereitungsgefäß (4) eingeführt ist, dass das die Probe aufweisende Ende des Probenentnahmestäbchens auf den Gefäßboden (9) aufsetzt. Das Aufbereitungsgefäß (4) weist eine Öffnung (5) auf, vertikal unterhalb der Öffnung befindet sich eine erste Kante (6). In dieser Figur nicht abgebildet vertikal auf gleicher Höhe wie die erste Kante rechts neben der ersten Kante befindet sich die Einführungsöffnung. Auf dem Gefäßboden befinden sich mehrere Stege (10), darunter ein Auslasskanal (13). Aus der Gefäßwand ragt über das die Probe aufweisende Ende des Probenentnahmestäbchens eine weitere Kante (14).
**Fig. 1b** zeigt die in **Fig. 1a** dargestellte nichterfindungsgemäße Vorrichtung, abgesehen davon, dass der Übersichtlichkeit halber das Probenentnahmestäbchen nicht mit abgebildet ist.

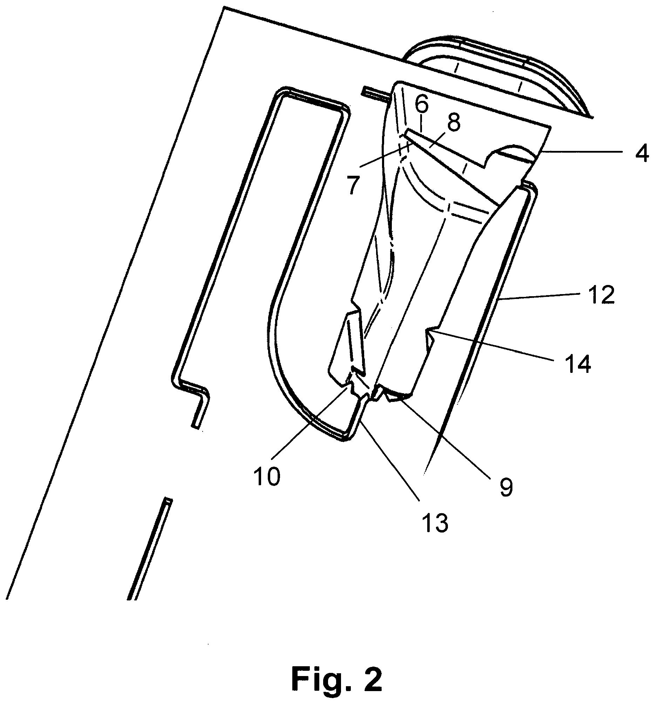

**Fig. 2** zeigt die in **Fig. 1a** dargestellte Vorrichtung, abgesehen davon, dass sie in ein Laborgerät, bevorzugt einen Laborchip integriert und gegenüber der Darstellung in **Fig. 1a** nach hinten gekippt ist.

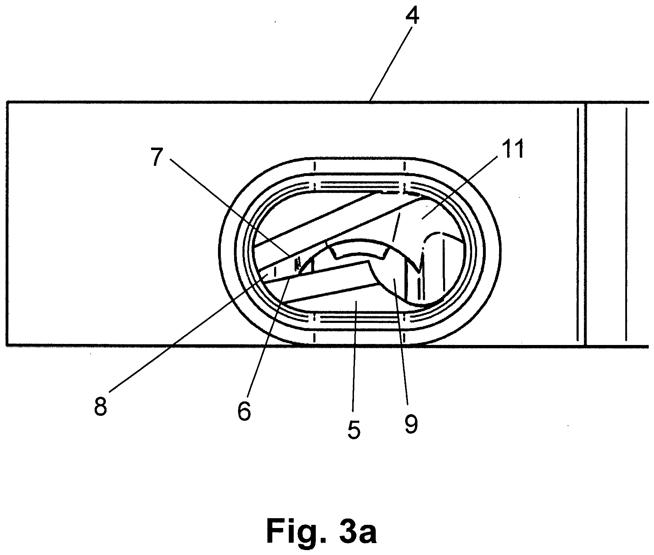

**Fig. 3a** zeigt eine erfindungsgemäße Vorrichtung, die senkrecht von oben gezeigt ist. Dadurch sind die erste Kante (6), die ihr auf gleicher Höhe gegenüber liegende zweite Kante (7), der von ihnen gebildete, zwischen ihnen befindliche Spalt (8) und die Einführungsöffnung (11) sichtbar. Darunter befindet sich der Gefäßboden (9).

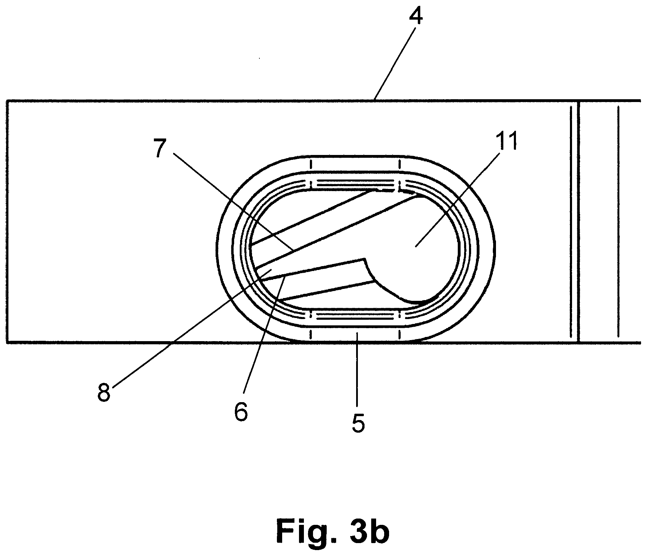

**Fig. 3b** ist die gleiche Abbildung wie **Fig. 3a****,** abgesehen davon, dass Merkmale der Übersichtlichkeit halber vertikal unterhalb der auf einer Ebene liegenden Einführungsöffnung (11), ersten Kante (6), zweiten Kante (7) und dem zwischen ihnen liegenden Spalt (8) ausgeblendet sind.

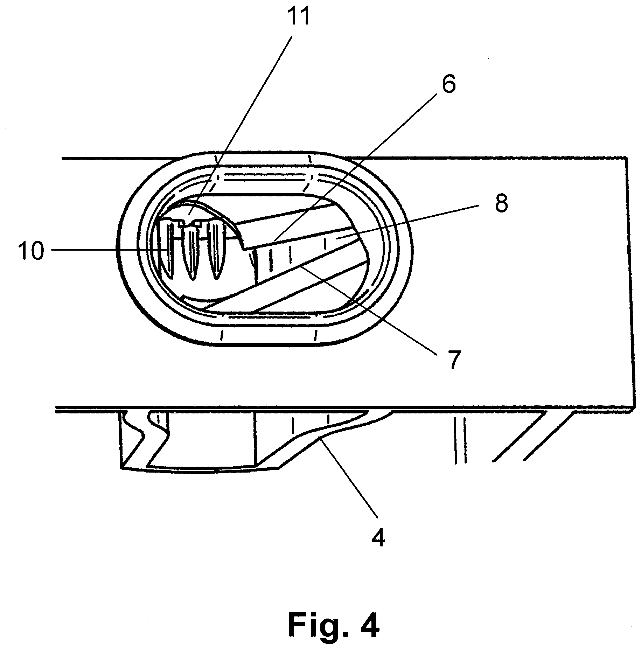

**Fig. 4** entspricht der **Fig. 3a****,** abgesehen davon, dass die erfindungsgemäße Vorrichtung mit der Öffnung nach hinten gekippt erscheint. Dadurch sind Stege (10) am Gefäßboden erkennbar.

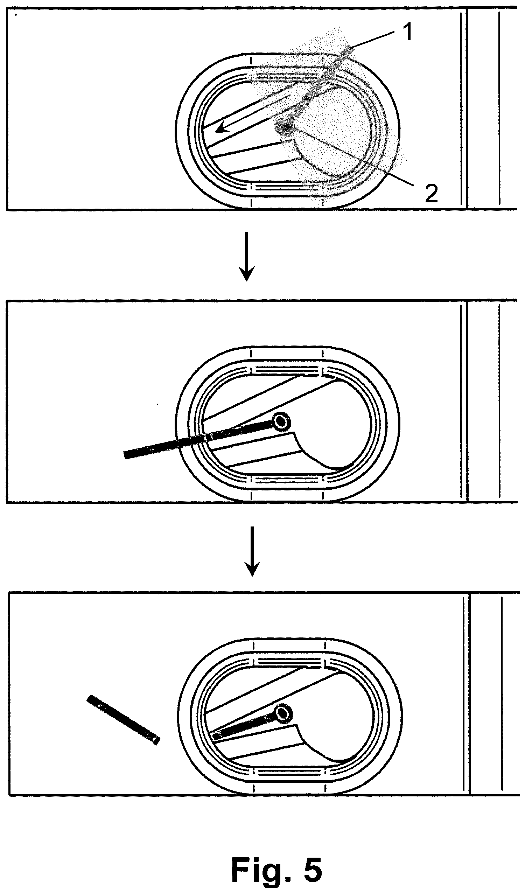

**Fig. 5** illustriert eine bevorzugte Ausführungsform des erfindungsgemäßen Verfahrens. Das Probenentnahmestäbchen wird durch die Einführöffnung in das erfindungsgemäße Aufbereitungsgefäß eingeführt, bis das Ende des Probenentnahmestäbchens auf den Gefäßboden aufsetzt. Anschließend wird es in den Spalt und weiter bis zum Ende des Spalts geschoben, wobei die Stelle am Gefäßboden, auf der das Ende Probenentnahmestäbchens mit der zu untersuchenden Probe aufsetzt, bevorzugt unverändert bleibt. Schließlich wird das Probenentnahmestäbchen abgebrochen.

### Bezugszeichenliste:

- 1: Probenentnahmestäbchen
- 2: Ende mit zu untersuchender Probe
- 3: Sollbruchstelle
- 4: Aufbereitungsgefäß
- 5: Öffnung
- 6: Erste Kante
- 7: Zweite Kante
- 8: Spalt
- 9: Gefäßboden
- 10: Steg
- 11: Einführungsöffnung
- 12: Einlasskanal
- 13: Auslasskanal
- 14: Weitere Kante

## Patentansprüche

1. Aufbereitungsgefäß umfassend einen Gefäßboden sowie eine Öffnung,
in die ein Probenentnahmestäbchen bis zum Gefäßboden eingeführt werden kann, wobei das Probenentnahmestäbchen ein Stäbchen umfasst, das durch mechanischen Druck an der Stelle abbricht, auf die der Druck ausgeübt wird, und wobei das Probenentnahmestäbchen an einem Ende des Stäbchens eine zu untersuchende Probe umfasst,
- wobei das Probenentnahmestäbchen mit dem die Probe aufweisenden Ende voran in Richtung des Gefäßbodens eingeführt werden kann, bis dieses Ende auf den Gefäßboden aufsetzt,
- wobei das Gefäß eine erste Kante aufweist,
- wobei das Aufbereitungsgefäß neben der ersten Kante eine zweite Kante aufweist, die vertikal auf gleicher Höhe wie die erste Kante angeordnet ist, so dass beide Kanten einen Spalt bilden,
- wobei sich der Spalt vom Inneren der Öffnung nach außen verengt,
- wobei der Gefäßboden einen oder mehr als einen Steg aufweist, über den bzw. die das in das Gefäß eingeführte und auf den Gefäßboden aufgesetzte Probenentnahmestäbchen derart abgebrochen werden kann, dass das unterhalb der Öffnung abgebrochene Stäbchen im Gefäß verbleibt und der Rest des Stäbchens entfernt werden kann.

2. Aufbereitungsgefäß nach Anspruch 1, wobei das Gefäß konisch ausgeführt ist.

3. Aufbereitungsgefäß nach einem der Ansprüche 1 bis 2, wobei die Kanten und der Spalt an eine Einführöffnung zum Einführen eines Endes eines Probenentnahmestäbchens anschließt,
wobei der Durchmesser der Einführöffnung an der breitesten Stelle wenigstens so groß, bevorzugt größer ist als der Durchmesser des Spaltes an dessen breitester Stelle und die Einführöffnung so ausgestaltet ist, dass das Probenentnahmestäbchen durch die Einführöffnung bis zum Gefäßboden in das Aufbereitungsgefäß eingeführt werden kann.

4. Aufbereitungsgefäß nach einem der Ansprüche 1 bis 3, wobei das Aufbereitungsgefäß einen Auslass- und/oder Einlasskanal aufweist und optional in ein Laborgerät, bevorzugt einen Laborchip integriert ist.

5. Vorrichtung umfassend
a) ein Aufbereitungsgefäß nach einem der Ansprüche 1 bis 4, und
b) ein Probenentnahmestäbchen umfassend ein Stäbchen mit einer Sollbruchstelle sowie eine Probe an einem Ende des Stäbchens.

6. Eine Vorrichtung nach Anspruch 5, wobei das Ende, an dem die Probe lokalisiert ist, mit einem probenaufnehmenden Material ausgestattet ist.

7. Eine Vorrichtung nach einem der Ansprüche 5 oder 6, wobei das Ende, an dem die Probe lokalisiert ist, verdickt ist.

8. Verfahren umfassend die Schritte
a) Einführen eines Probenentnahmestäbchens, aufweisend eine zu untersuchende Probe in das Aufbereitungsgefäß nach einem der Anspruche 1 bis 4, bis das die Probe aufweisende Ende des Probenentnahmestäbchen auf den Gefäßboden aufsetzt,
b) Abbrechen des Probenentnahmestäbchens,
c) optional Verschließen des Aufbereitungsgefäßes und
d) Kontaktieren des Endes des Probenentnahmestäbchens, an dem sich die zu untersuchende Probe befindet, mit einer Flüssigkeit unter Freisetzung der Probe die Flüssigkeit.

9. Verfahren gemäß Anspruch 8,
wobei das Aufbereitungsgefäß ein Aufbereitungsgefäß nach einem der Ansprüche 1 bis 4 ist und das Probenentnahmestäbchen in Schritt a) zum nachfolgenden Abbrechen bis zur engsten Stelle des Spalts geführt wird.

## Claims

1. Preparation vessel comprising a vessel base and an opening, into which opening a sampling rod can be inserted as far as the vessel base, wherein the sampling rod comprises a rod which, by means of mechanical pressure, breaks off at the place where the pressure is exerted, and wherein the sampling rod comprises, at one end of the rod, a sample that is to be tested,
- wherein the sampling rod can be inserted, with the end having the sample to the front, in the direction of the vessel base, until this end sits on the vessel base,
- wherein the vessel has a first edge,
- wherein the preparation vessel has, in addition to the first edge, a second edge which is arranged vertically at the same height as the first edge, such that both edges form a gap,
- wherein the gap narrows outwards from the interior of the opening,
- wherein the vessel base has one or more than one web, via which the sampling rod inserted into the vessel and placed onto the vessel base can be broken off in such a way that the rod broken off below the opening remains in the vessel and the rest of the rod can be removed.

2. Preparation vessel according to Claim 1, wherein the vessel is configured conically.

3. Preparation vessel according to either of Claims 1 and 2, wherein the edges and the gap adjoin an insertion opening for the insertion of an end of a sampling rod,
wherein the diameter of the insertion opening at the widest point is at least so great, preferably greater than the diameter of the gap at its widest point, and the insertion opening is so configured that the sampling rod can be inserted through the insertion opening into the preparation vessel as far as the vessel base.

4. Preparation vessel according to one of Claims 1 to 3, wherein the preparation vessel has an outlet and/or inlet channel and is optionally integrated in a laboratory appliance, preferably a laboratory chip.

5. Device comprising
a) a preparation vessel according to one of Claims 1 to 4, and
b) a sampling rod comprising a rod with a predetermined breaking point and a sample at one end of the rod.

6. Device according to Claim 5, wherein the end at which the sample is located is equipped with a sample-receiving material.

7. Device according to either of Claims 5 and 6, wherein the end at which the sample is located is thickened.

8. Method comprising the steps of:
a) inserting a sampling rod, which has a sample to be tested, into the preparation vessel according to one of Claims 1 to 4, until the end of the sampling rod having the sample sits on the vessel base,
b) breaking off the sampling rod,
c) optionally closing the preparation vessel, and
d) bringing the end of the sampling rod at which the sample to be tested is located into contact with a liquid, with release of the sample into the liquid.

9. Method according to Claim 8, wherein the preparation vessel is a preparation vessel according to one of Claims 1 to 4, and the sampling rod is guided in step a) to the narrowest point of the gap in order then to be broken off.

## Revendications

1. Récipient de préparation, comprenant un fond de récipient ainsi qu'une ouverture dans laquelle un bâtonnet de prélèvement d'échantillon peut être introduit jusqu'au fond de récipient, le bâtonnet de prélèvement d'échantillon comprenant un bâtonnet qui se casse par pression mécanique à l'endroit auquel la pression est exercée, et le bâtonnet de prélèvement d'échantillon comprenant, à une extrémité du bâtonnet, un échantillon à examiner,
- le bâtonnet de prélèvement d'échantillon pouvant être introduit avec l'extrémité présentant l'échantillon en premier dans la direction du fond de récipient jusqu'à ce que cette extrémité repose sur le fond de récipient,
- le récipient présentant une première arête,
- le récipient de préparation présentant en plus de la première arête, une deuxième arête qui est disposée verticalement à la même hauteur que la première arête de telle sorte que les deux arêtes forment une fente,
- la fente se rétrécissant vers l'extérieur depuis l'intérieur de l'ouverture,
- le fond de récipient présentant une ou plus d'une nervure par le biais de laquelle ou desquelles le bâtonnet de prélèvement d'échantillon introduit dans le récipient et posé sur le fond de récipient peut être cassé de telle sorte que le bâtonnet cassé en dessous de l'ouverture reste dans le récipient et que le reste du bâtonnet puisse être enlevé.

2. Récipient de préparation selon la revendication 1, le récipient étant réalisé sous forme conique.

3. Récipient de préparation selon l'une quelconque des revendications 1 et 2, dans lequel les arêtes et la fente se raccordent au niveau d'une ouverture d'introduction pour introduire une extrémité d'un bâtonnet de prélèvement d'échantillon,
le diamètre de l'ouverture d'introduction à l'endroit le plus large étant au moins aussi grand, de préférence étant plus grand, que le diamètre de la fente à son endroit le plus large et l'ouverture d'introduction étant configurée de telle sorte que le bâtonnet de prélèvement d'échantillon puisse être introduit à travers l'ouverture d'introduction jusqu'au fond de récipient dans le récipient de préparation.

4. Récipient de préparation selon l'une quelconque des revendications 1 à 3, le récipient de préparation présentant un conduit de sortie et/ou un conduit d'entrée et étant facultativement intégré dans un appareil de laboratoire, de préférence un laboratoire sur puce.

5. Dispositif comprenant
a) un récipient de préparation selon l'une quelconque des revendications 1 à 4, et
b) un bâtonnet de prélèvement d'échantillon comprenant un bâtonnet avec une zone destinée à la rupture ainsi qu'un échantillon à une extrémité du bâtonnet.

6. Dispositif selon la revendication 5, dans lequel l'extrémité à laquelle se trouve l'échantillon est munie d'un matériau recevant l'échantillon.

7. Dispositif selon l'une quelconque des revendications 5 ou 6, dans lequel l'extrémité à laquelle se trouve l'échantillon est épaissie.

8. Procédé comprenant les étapes consistant à
a) introduire un bâtonnet de prélèvement d'échantillon présentant un échantillon à examiner dans le récipient de préparation selon l'une quelconque des revendications 1 à 4, jusqu'à ce que l'extrémité du bâtonnet de prélèvement d'échantillon présentant l'échantillon repose sur le fond de récipient,
b) rompre le bâtonnet de prélèvement d'échantillon,
c) facultativement, fermer le récipient de préparation et
d) mettre en contact l'extrémité du bâtonnet de prélèvement d'échantillon à laquelle se trouve l'échantillon à examiner avec un liquide, en libérant de l'échantillon dans le liquide.

9. Procédé selon la revendication 8,
dans lequel le récipient de préparation est un récipient de préparation selon l'une quelconque des revendications 1 à 4, et le bâtonnet de prélèvement d'échantillon est guidé à l'étape a) jusque qu'au point le plus étroit de la fente en vue de sa rupture subséquente.
